# FASCICULE DE BREVET EUROPEEN

(11) **EP 0 680 984 B1**
(45) Date de publication et mention de la délivrance du brevet: **08.03.2000**
(21) Numéro de dépôt: 95400934.6
(22) Date de dépôt: 26.04.1995
(51) Int. Cl.: C08G 18/80

(54) **Isocyanates masqués au moyen de composés hydroxyaromatiques**
Mit Hydroxyaromatverbindungen blockierte Isocyanate
Isocyanates blocked with hydroxyaromatic compounds

(30) Priorité: 04.05.1994 FR 9405436
(43) Date de publication de la demande: 08.11.1995
(60) Demande divisionnaire: 99112178.1
(73) Titulaire: RHODIA CHIMIE, 92408 Courbevoie Cédex (FR)
(72) Inventeur: Ardaud, Pierre, F-69110 Sainte-Foy-Les-Lyon (FR); Bernard, Jean-Marie, Saint-Laurent d'Agny, F-69440 Mornant (FR)
(74) Mandataire: Ricalens, François

(56) Documents cités:
- EP-A- 0 214 495
- EP-A- 0 562 394
- FR-A- 2 266 725
- FR-A- 2 370 764
- US-A- 3 317 463

## Description

La présente invention a pour objet une nouvelle famille d'isocyanates masqués. Elle concerne plus particulièrement des isocyanates masqués au moyen de dérivés hydroxyaromatiques, et leur utilisation dans les techniques de revêtement au moyen de poudres.

Pour des raisons liées à la protection de l'environnement et à la sécurité du travail, on cherche à éliminer de plus en plus dans les techniques de revêtement, et notamment de peinture, l'utilisation des solvants.

Dans ce contexte, des techniques de revêtement au moyen de poudres se développent de plus en plus.

Les isocyanates masqués commencent à être utilisés mais leur emploi est limité par le peu de composés répondant aux exigences de la chimie des poudres.

Une première difficulté réside dans la difficulté à trouver des isocyanates ou mélanges d'isocyanates masqués qui restent sous forme de poudre dans les conditions de stockage usuelles, conditions qui peuvent varier beaucoup d'un endroit à un autre. Cela implique que ces composés aient un point de fusion et/ou de transition vitreuse (Tg) relativement élevé.
les dérivés, objet de la présente étude, n'ont pas toujours un point de fusion franc, aussi dans ce cas détermine-t-on un point de fusion apparent soit au banc koffler soit à l'aide d'une méthode de type dite capillaire (par exemple point de fusion dit "de büchi"). Un point de transition vitreuse peut être mesuré par les techniques d'analyse thermique différentielle (ATD).

Il faut également que ces composés aient des points de transition vitreuse et des points de fusion suffisamment bas pour qu'ils puissent réagir dans les conditions d'utilisation des poudres.

Il faut en outre que les composés issus des réactions de réticulation ne soient délétères ni pour la santé humaine ou animale, ni pour l'environnement.

FR 2 266 725 décrit une composition de résine polyester en poudre pour peinture pulvérulente, obtenue par mélange d'un polyester à l'état fondu avec un polyisocyanate bloqué par un p-hydroxybenzoate d'alkyle, dans lequel la fraction alkyle contient moins de 5 atomes de carbone, le polyester étant lui-même obtenu par dépolymérisation d'un polyester de haut degré de polymérisation et de viscosité intrinsèque égale ou supérieure à 0,4, par un alcool non volatil ou un ester présent au moins un groupe hydroxyalcoolique, le polyester fondant à une température comprise entre 45 et 120°C, et présentant des groupes hydroxy terminaux ainsi qu'un degré moyen de polymérisation compris entre environ 5 et environ 50.

EP 0 562 394 décrit une préparation poudre de polyuréthane expansible contenant un agent d'expansion obtenue en faisant réagir une émulsion d'un composé comprenant des fonctions NCO libres ou réagissant avec des fonctions NCO libres avec un composé comportant 2 atomes d'hydrogène réagissant avec la fonction NCO libre ou réciproquement comportant deux groupes NCO libres, la réaction pouvant avoir lieu en présence d'un composé comportant une partie des groupes NCO bloqués.
Le composé comportant des groupes NCO en partie bloqués est le produit de réaction de l'isocyanurate du 1,6-hexaméthylène diisocyanate avec un ester d'acide hydroxybenzoïque ainsi qu'un polyol de masse moléculaire 400-60 000 ou est un polyisocyanate comprenant des groupes uretdione ou un produit de réaction de ce composé polyisocyanate comprenant des groupes uretdione et d'un ou plusieurs composés comprenant des fonctions réactives vis à vis de NCO.

Un des buts de la présente invention est de fournir une nouvelle famille d'isocyanates bloqués qui réponde aux contraintes évoquées ci-dessus.

Un autre but de la présente invention est de fournir des compositions utilisables dans le revêtement au moyen de poudres et qui contiennent des isocyanates bloqués.

Un autre but de la présente invention est de fournir un procédé de synthèse des isocyanates répondant aux contraintes ci-dessus.
Un autre but de la présente invention est de fournir un procédé d'utilisation des isocyanates masqués selon la présente invention et répondant aux contraintes ci-dessus.

Selon la présente invention, ces buts sont atteints au moyen d'isocyanates masqués. purs ou en mélange, et qui sont issus de la condensation d'un dérivé aromatique hydroxylé sur le noyau et portant une fonction choisie parmi les fonctions nitriles et de préférence carbonyles avec un isocyanate comme décrit dans la revendication 1.

Il convient de choisir parmi les membres de cette famille ceux pour lesquels il est possible de déterminer un point de fusion apparent, cette mesure étant réalisée à température ambiante (20°C). Ce point de fusion doit être au moins égal à 30°C (un chiffre significatif), avantageusement à 50°C.
Il est souhaitable que les produits ne mottent pas. Donc on choisit des composés qui broyés, et stockés à température ambiante présentent une granulométrie similaire à 24 heures d'intervalle.
Le caractère mottant est en général peu ou prou lié au point de transition vitreuse (Tg). Ainsi, les composés préférés sont ceux qui présentent un point de transition vitreuse (Tg) au moins égal à 10°C (deux chiffre significatifs), avantageusement à 20°C (un chiffre significatif, de préférence deux), de préférence à 30°C ( deux chiffres significatifs).

Ainsi, parmi les parahydroxybenzoates d'alcoyle, le choix des alcoyles peut être critique : les alcoyles de plus de deux carbones qui sont linéaires sont soit de point de fusion insuffisamment élevé, soit sont mellifluents en cristallisant seulement au bout dune durée longue allant d'une semaine à plusieurs mois ce qui les rend difficile d'usage et donc ne sont pas préférés ainsi le n-propyle, le n-butyle et plus généralement les n-alcoyle sont d'usage difficiles. En outre, les chaînes longues pour des raisons similaires sont aussi à éviter, notamment celles dont le nombre de carbone est supérieur à 6.

Le cas de l'éthyle est intermédiaire et donne des résultats acceptables mais non excellents. L'isopropyle et surtout le méthyle sont préférés.
Les fonctions nitriles et de préférence carbonyles peuvent être liées au noyau soit par une simple liaison, soit par l'intermédiaire d'un chaînon lequel peut être chalcogène, azote ou phosphore porteur d'un hydrogène ou d'un substituant ou méthylène éventuellement substitué.
Compte tenu du fait que l'effet électroattracteur diminue ou disparaît par l'intercalation d'un chaînon entre le groupe électroattracteur et le noyau, la liaison directe entre le noyau et le groupe électroattracteur est préférée, à moins qu'il n'y ait déjà un groupe électroattracteur sur le noyau, ou que celui-ci soit naturellement pauvre en électrons (par exemple hétérocycle à six chaînons).
Les chaînons chalcogènes ou uniquement porteurs d'hydrogène et/ou dans une moindre mesure de méthyle sont préférés. Les chaînons à base d'élément(s) de la deuxième ligne (ligne de l'oxygène) du tableau périodique des éléments sont préférés.
Selon la présente invention, l'isocyanate masqué, pur ou en mélange, est issu d'un polyisocyanate, c'est-à-dire possédant au moins deux fonctions isocyanates, avantageusement plus de deux, (possibilité de valeurs fractionnaires puisqu'il s'agit en général de mélange d'oligomères plus ou moins condensés) lequel est lui-même le plus souvent issu d'une précondensation ou d'une prépolymérisation de diisocyanate unitaire.
D'une manière générale, les masses moléculaires moyennes de ces prépolymères ou de ces précondensats sont au plus égales à 2000 (un chiffre significatif), plus couramment à 1000 (un chiffre significatif de préférence deux).

Ainsi, parmi les polyisocyanates utilisés pour l'invention, on peut citer ceux de type biuret et ceux dont la réaction de di- ou trimérisation a conduit à des cycles à quatre, cinq ou six chaînons. Parmi les cycles à six chaînons, on peux citer les cycles isocyanurique issus d'une homo- ou d'une hétéro-trimérisation de divers diisocyanates seuls, avec d'autres isocyanate(s) [mono-, di--, ou poly-isocyanate(s)] ou avec du gaz carbonique (ou bioxyde de carbone). Dans ce cas, on remplace un azote du cycle isocyanurique par un oxygène.
Les polyisocyanates préférés sont ceux qui présentent au moins une fonction isocyanate aliphatique. En d'autres termes, au moins une fonction isocyanate masquée selon l'invention est reliée au squelette par l'intermédiaire d'un carbone de type SP3 portant avantageusement un atome d'hydrogène, de préférence deux.
Le dérivé aromatique hydroxylé sur le noyau, servant à masquer la fonction isocyanate est avantageusement choisi parmi ceux de formule (I) :

Ar(R)ₙ(Y-Z)ₘ(OH)ₚ (I)

où Ar est un reste aromatique sur lequel est greffé n substituants R, m fonctions polaires Z choisies parmi les nitriles et les groupements carbonyles, et p fonctions hydroxyles.

Les valeurs de n, m et p sont telles que la somme n + m + p soit au plus égale au nombre de sommets substituables, avantageusement p est au plus égal à 2, de préférence p est égal à 1.
Avantageusement, m est au plus égal à deux, de préférence m est égal à 1.
Avantageusement n est au plus égal à 3, de préférence choisi parmi zéro, un, et deux, plus préférentiellement égal à zéro.
R représente des substituants indifférents pour la réaction de masquage et en général correspond à des chaînes hydrocarbonése, le plus souvent des chaînes alcoyles au sens étymologique du terme, à savoir un alcool auquel on a enlevé sa fonction hydroxyle.

Deux substituants vicinaux R peuvent être reliés entre eux pour former un cycle, qui peut être aromatique par exemple .

Z est avantageusement choisi parmi les groupements présentant une fonction carbonyle. Parmi ces fonctions, il convient de citer les fonctions alcoxycarbonyles (ou en d'autres termes les fonctions esters), la fonction amide, la fonction cétone avec la condition préférentielle qu'il n'y ait pas d'hydrogène acide (en d'autres termes la fonction n'est avantageusement pas porteuse d'hydrogène ou si elle en porte, le pKa correspondant est au moins égal à environ 20 (un chiffre significatif, de préférence deux) plus préférentiellement au moins égal à environ 25 en a de la fonction carbonyle (ester, cétone ou amide). Ainsi, les amides (y compris lactame, voire urée) préférés sont avantageusement substitués, de préférence suffisamment pour qu'il n'y ait pas d'hydrogène sur l'azote de la fonction amide ou de manière qu'il n'y ait pas d'hydrogène réactif.
où Y est choisi parmi les groupements divalents
avantageusement -O-, -S-, NR'-,-CR'R"- avec R' et R" choisi parmi les radicaux hydrocarbonés, avantageusement alcoyle, de 1 à 6 atomes de carbone, avantageusement de 1 à 4, de préférence méthyle, plus préférentiellement hydrogène et de préférence Y représente une liaison simple.
Il est préférable que la ou les fonctions polaires Z (en général choisies parmi la fonction nitrile et/ou les fonctions carbonyles) ne soient pas vicinales du groupe Z comme par exemple dans l'acide salicylique.
Le reste aromatique Ar est constitué d'un ou plusieurs noyaux avantageusement condensés, hétéro- ou homocycliques. Il est préférable que Ar ne comporte pas plus de deux noyaux, et de préférence pas plus d'un noyau.
Le reste aromatique Ar peut être constitué d'un ou plusieurs noyaux hétéro- ou homocycliques. le plus souvent homocyclique en raison de leur facilité d'accès. Il convient toutefois de souligner l'intérêt des hétérocycles à 6 chaînons qui présente une température de libération très inférieure à celle des homocycles correspondants.
Il est souhaitable que le nombre total de carbones du dérivé aromatique hydroxylé sur le noyau soit au plus égal à 20, de préférence à 10 (un chiffre significatif).
Ce noyau est avantageusement à 6 chaînons, les chaînons étant constitués de carbone ou d'azote avec le nombre de substituants nécessaires à la valence de ces atomes.
Parmi les acides donnant les résultats les plus satisfaisants, il convient de citer les acides greffés sur un noyau benzénique ou sur des noyaux pyridiniques. Ainsi, les acides métahydroxy- ou parahydroxybenzoïques et surtout leurs esters donnent de bons résultats.
Comme cela a déjà été mentionné, selon la présente invention, il est préférable que le point de fusion du composé ou du mélange de composés obtenu présente un point de fusion apparent au moins égal à 30°C, de préférence 50°C.

Il est également préférable que la température de transition vitreuse soit au moins égale à 20°C, avantageusement à 40°C.

Il est préférable de choisir les composés selon la présente invention de manière à ce qu'ils réagissent complètement avec un alcool primaire à 250°C en moins d'une demi-heure.
On considère que la réaction est complète si elle est réalisée à 90 % ou plus.
Les isocyanates pour lesquels l'invention est la plus intéressante sont ceux dont l'atome d'azote est lié à un carbone d'hybridation sp³ et plus particulièrement aux isocyanates aliphatiques, et notamment aux polyméthylènes diisocyanates et leurs différents dérivés de condensation (biuret, etc.....) et de di- et de "trimérisation".
Selon la présente invention, II est souhaitable et parfois nécessaire que le pourcentage de fonction isocyanate libre résiduelle soit au plus égal à 5 %, avantageusement à 3 %, de préférence à 1 %. Les points de fusion ou de transition vitreuse les plus élevés sont obtenus avec des pourcentages ne dépassant pas 0,5 %. Les teneurs en dérivé aromatique hydroxylé sur le noyau sont également avantageusement faibles, c'est-à-dire au plus égales à 5 %, avantageusement à 3 %, de préférence à 1 %.
Comme cela a été mentionné au début de la présente description, la présente invention vise également des compositions de poudres qui comportent un polyisocyanate masqué ou un mélange de polyisocyanates masqués, selon la présente invention.
Dans la présente description, les caractéristiques de granulométrie font souvent référence à des notations du type dₙ où n est un nombre de 1 à 99, cette notation est bien connue dans de nombreux domaines techniques, mais est un peu plus rare en chimie. Aussi, peut-il être utile d'en rappeler la signification. Cette notation représente la taille de particule telle que n % (en poids, ou plus exactement en masse, puisque le poids n'est pas une quantité de matière mais une force) des particules soit inférieur ou égal à la dite taille.
Dans les compositions de poudre selon cette mise en oeuvre, les isocyanates masqués selon la présente invention, constituent avantageusement une population (avantageusement distincte de celle des coréactifs) de particules dont le d₉₀ est au plus égal à 200 microns, avantageusement à 100 microns, de préférence à 50 microns. Cette population de particules présente un d₁₀ au moins égal à 1 micron, avantageusement à 5 microns, de préférence à 10 microns.

Les compositions des poudres comportent avantageusement au moins un polyol (au moins diol) ou, dans certains cas, des polyamines. On peut également avoir des composés polyfonctionnels présentant au moins deux fonctions choisies parmi les fonctions amines ou -ols (phénols ou de préférence alcools). Les composés ci-dessus peuvent présenter en outre d'autre fonctions (par exemple fonction acide telle que carboxylique ou sulfonique) à condition que ces fonctions n'empêchent pas la condensation ou la réticulation.

Ces polyols ou polyamines forment également eux-mêmes des poudres et répondent aux mêmes contraintes de point de fusion et de température de transition vitreuse que celles évoquées précédemment.
Il est préférable que le point de fusion des compositions selon la présente invention présente un point de fusion au moins égal à 50°C. Il est même souhaitable que les températures de ramollissement soit telles qu'il n'y ait pas frittage de la poudre à une température d'au moins 50°C.
Il est également préférable que leur température de transition vitreuse soit au moins égale à 40°C.
Avantageusement, les compositions de poudres comportent également au moins un catalyseur, en général et de préférence des catalyseurs à base d'étain ou de zinc.
Le cas échéant, elles comportent des additifs usuels en la matière, tels que des charges, des pigments ( TiO₂... ) des additifs d'amélioration des propriétés physiques (tension superficielle, résistance au vieillissement, à la lumière, facilité de mise en oeuvre...)
Selon la présente invention, un procédé de synthèse consiste à mettre en contact dans un solvant l'isocyanate libre, ou partiellement libre, avec le composé hydroxyaromatique, c'est-à-dire le composé de type phénolique.
Lorsque les composés selon l'invention et leur précurseurs sont stables dans les conditions ci-après, on peut réaliser la synthèse sans solvant mais à l'état fondu. Le produit final est alors refroidi, par exemple en fabriquant des écailles, qui peuvent être obtenues par le refroidissement brutal occasionné en versant le mélange réactionnel sur une paroi froide. Les écailles obtenues peuvent être broyées.
Il est important pour obtenir de bons (c'est-à-dire de faibles) pourcentages de fonction isocyanate libre résiduelle, d'introduire le dérivé aromatique hydroxylé sur le noyau en quantité très proche de la stoechiométrie. Il est préférable d'être en excès stoechiométrique léger (de 0,5 à 2%, de préférence au plus 1 %).
Il est également préférable d'ajouter un catalyseur de condensation des isocyanates sur des fonctions -ol. Ces catalyseur de condensation sont souvent à base d'étain ou d'amine tertiaire.
La température de fin de condensation est avantageusement au plus égale à 100°C (un chiffre significatif de préférence deux) de préférence à 80°C et avantageusement au moins égale 50°C, de préférence à 60°C. En effet si l'on chauffe trop, le pourcentage d'isocyanate libre risque d'être trop élevé.
Lorsqu'il y a solvant, il est de préférence choisi de manière à être suffisamment polaire pour dissoudre au moins 50, de préférence au moins 100, préférentiellement au moins 200 grammes par litre de l'isocyanate initial.
Une fois la réaction terminée, il convient de précipiter le produit final, soit selon une technique classique de cristallisation, et plus préférentiellement par addition d'un composé précipitant suffisamment peu polaire pour provoquer la précipitation sans qu'il y ait nécessairement cristallisation.
Le composé précipitant est bien évidemment un composé de type volatil, et le plus souvent des composés du type mélange d'hydrocarbures légers, du type éther de pétrole ou du type hexane ou heptane. On peut également utiliser, seuls ou en mélange, les éthers d'alcool léger (c'est à dire les alcools comportant au plus six atomes de carbones de préférence 4).
Généralement, on utilise des composés du type alcane ou alcène dont le nombre de carbones est inférieur à 20 et supérieur à 4.

Les exemples non limitatifs suivants illustrent l'invention.

### Exemple 1

### Synthèse du Tolonate HDT bloqué p-Hydroxy benzoate de méthyle :

Dans un réacteur de 500 ml, charger :
- trimère de l'hexaméthylène diisocyanate vendu sous la marque Tolonate HDT® = 54,2 g (indice NCO = 22,1 %)
- Sovesso 100® = 25g.

Sous agitation et à température ambiante, ajouter en plusieurs fois.
- p-Hydroxy benzoate de méthyle = 47,6 g (0,31 mole).

La masse réactionnelle est chauffée jusqu'à 60°C et maintenue à cette température jusqu'à disparition des fonctions NCO.

Après refroidissement, le produit désiré (polyisocyanate bloqué) précipite. Il est réduit en poudre et lavé au moyen de n-hexane.
- n Hexane = 41,2 g.

La masse réactionnelle est filtrée, le solide obtenu lavé par plusieurs fractions d'hexane puis broyé et séché à nouveau.
- masse obtenue = 95,7 g
- point de fusion = 85°C.
   RMN on remarque 3 pics à
   7,8. ppm (hydrogène porté par l'azote du carbamate)
   7,9. ppm (hydrogène aromatique en ortho de la fonction ester carbamique)
   7,10 ppm (hydrogène aromatique en ortho de la fonction carbonyle)

### Exemple 2

### Formulation vernis du produit précédent :

Le produit intermédiaire obtenu (I) est formulé de la façon suivante :
- I = 6,0 g
- Desmophen 690® = 14,0 g (% OH = 2 %)
Soit un rapport NCO/OH = 1

Le mélange des deux poudres est broyé jusqu'à obtenir un mélange parfaitement homogène d'une granulométrie inférieure à 50 µm.

Une fraction de cette poudre est appliquée en couche d'épaisseur 300 µm sur plaque acier et traitée thermiquement à différentes températures pendant 30 ou 60 minutes.

| **CUISSON** | **30 minutes** | | **60 minutes** | |
|---|---|---|---|---|
| | Test Solvant (2) | Dureté à chaud (1) | Test solvant (2) | Dureté à chaud (1) |
| 130°C | D | M | D | M |
| 160°C | D | M | D | M |
| 190°C | D | M | I | TB |
| 200°C | I | TB | I | TB |

Le film obtenu est qualifié par sa dureté et sa résistance au solvant :
(1) TB = Très bonne : M = Médiocre
(2) Dépôt d'une goutte de Méthyl - Ethyl Cétone et observation de la détérioration du film,
   D = le film est dégradé par l'action du solvant
   I = le film est intact après action du solvant

### Mode Opératoire :

### Exemple 3 (exemple comparatif)

Introduire dans un réacteur les produits suivants :
- Tolonate HDT ® : 100 grs ( 0.529 mole NCO )
Ajouter
- p Hydroxy benzoate de Méthyle : 81.3 grs ( 0.529 mole )

Chauffer sous agitation jusqu'à obtenir la fusion de l'agent bloquant vers 85 °C , vers 100 °C le milieu est totalement limpide et incolore.
Chauffer à 120 °C et maintenir 1 h à cette température.
Après refroidissement, le produit se présente sous la forme d'une gomme dure légèrement collante
Tg = 8 °C on détermine par dosage à la dibutylamine la teneur en fonction NCO libre est d'environ 10 %

### Exemple 4

Introduire dans un réacteur les produits suivants :
- Tolonate HDT ® : 100 grs ( 0.529 mole NCO )
Ajouter - p Hydroxy benzoate de Méthyle : 81.3 grs ( 0.529 mole )
- Triéthyl amine ( TEA) : 0.2 grs

Chauffer sous agitation jusqu'à obtenir la fusion de l'agent bloquant vers 85 °C , vers 100 °C le milieu est totalement limpide et incolore.
Après maintien à 100 °C, on mesure par dosage à la dibutyl-amine la teneur en fonctions NCO libres.

| | | |
|---|---|---|
| 1 h à 100 °C | NCO libres= | 6.2 % |
| 2 h à 100 °C | NCO | 5.6 % |
| 5 h à 100 °C | NCO | 5.6 % |

### Exemple 5

Introduire dans un réacteur les produits suivants :
- Tolonate HDT ® : 100 grs ( 0.529 mole NCO )
Ajouter - p Hydroxy benzoate de Méthyle : 81.3 grs ( 0.529 mole )
- Triéthyl amine ( TEA) : 0.2 grs

Chauffer sous agitation jusqu'à obtenir la fusion de l'agent bloquant vers 85 °C , vers 100 °C le milieu est totalement limpide et incolore.
La masse réactionnelle est ensuite refroidie progressivement à 60 °C puis maintenue à cette température .

On mesure par dosage à la dibutyl-amine la teneur en fonctions NCO libres.

| | | |
|---|---|---|
| 30 min à 60 °C | NCO libres= | 3.1 % |
| 1 h à 60 °C | NCO | 2.6 % |
| 4 h à 60 °C | NCO | 2.6 % |

### Exemple 6

Introduire dans un réacteur les produits suivants :
- Tolonate HDT ® : 100 grs ( 0.529 mole NCO )
Ajouter - p Hydroxy benzoate de Méthyle : 81.3 grs ( 0.529 mole )
- Triéthyl amine ( TEA) : 0.5 grs
Chauffer sous agitation jusqu'à obtenir la fusion de l'agent bloquant vers 85 °C , vers 100 °C le milieu est totalement limpide et incolore.
La masse réactionnelle est ensuite refroidie progressivement à 60 °C puis maintenue à cette température .
On mesure par dosage à la dibutyl-amine la teneur en fonctions NCO libres.

| | | |
|---|---|---|
| 3 h à 60 °C : | NCO libres= | 1.2 % |

On rajoute dans le milieu réactionnel à nouveau 0.5 g de TEA puis après un nouveau maintien, on dose les fonctions NCO libres :

| | | |
|---|---|---|
| 3 h à 60 °C : | NCO | 0.2 % |

Après refroidissement, le produit se présente sous la forme d'une gomme dure qui peut être broyée.
Tg = 24 °C

### Exemple 7 (exemple comparatif)

Mode opératoire identique à l'exemple 4 mais en utilisant à la place du p Hydroxybenzoate de méthyle, le produit suivant :
p-Hydroxybenzoate d'Ethyle : 88.9 g (0.529 mole ) Après refroidissement, le produit se présente sous la forme d'une gomme solide
Tg = 18.8 °C

### Exemple 8 (exemple comparatif)

Mode opératoire identique à l'exemple 4 mais en utilisant à la place du p Hydroxybenzoate de méthyle, le produit suivant :
p-Hydroxybenzoate de Butyle : 77.8 g ( 0.529 mole ) Après refroidissement, le produit se présente sous la forme d'une liquide vitreux.
Tg = 5.5 °C

### Exemple 9

Mode opératoire identique à l'exemple 4 mais en utilisant à la place du p Hydroxybenzoate de méthyle, le produit suivant :
p-Hydroxybenzoate d'Isopropyle : 73.1 g ( 0.529 mole ) Après refroidissement, le produit se présente sous la forme d'une gomme solide.
Tg = 23 °C

### Exemple 10

### Formulation vernis du produit précédent :

Le produit intermédiaire obtenu (I)Tolonate HDT bloqué p-Hydroxy benzoate de Méthyle est formulé de la façon suivante :
- I = 38.3 g
- Johnson 587 ® = 61.7 g (% OH = 2.8 %)
Soit un rapport NCO/OH = 1.1

Le mélange des deux poudres est broyé jusqu'à obtenir un mélange parfaitement homogène d'une granulométrie inférieure à 50 µm.

Une fraction de cette poudre est appliquée en couche d'épaisseur 200 µm sur plaque acier et traitée thermiquement à différentes températures pendant 30 minutes.

| **CUISSON** | **30 minutes** | |
|---|---|---|
| | Test Solvant (2) | Dureté à chaud (1) |
| 130°C | D | M |
| 140°C | D | M |
| 150°C | D | M |
| 160°C | I | TB |

Le film obtenu est qualifié par sa dureté et sa résistance au solvant :
(1) TB = Très bonne : M = Médiocre
(2) Dépôt d'une goutte de Méthyl - Ethyl Cétone et observation de la détérioration du film,
   D = le film est dégradé par l'action du solvant
   I = le film est intact après action du solvant

## Revendications

1. Polyisocyanate masqué dont le point de fusion ou le point de fusion apparent dans le cas des composés n'ayant pas un point de fusion franc, mesuré au banc koffler ou à l'aide d'une méthode de type capillaire est au moins égal à 30°C, et le point de transition vitreuse au moins égal à 10°C,
caractérisé par le fait qu'il peut être obtenu par la condensation en excès stoechiométrique de 0,5 à 2 %,
• d'un dérivé aromatique hydroxylé sur le noyau et portant une fonction électro-attractrice choisie parmi les fonctions carbonyles et nitriles, avec la condition qu'il y ait liaison directe entre le noyau et la fonction électro-attractrice. à moins qu'il n'y ait déjà un groupe électro-attracteur sur le noyau, ou que celui-ci soit hétérocyclique à six chaînons, et avec la condition que lorsque la fonction carbonyle est une fonction ester sa partie alcoyle présente un nombre d'atomes de carbone au plus égal à 6 et est ramifiée quand son nombre de carbone excède 2 ;
• avec un polyisocyanate présentant plus de deux fonctions isocyanates, issu d'une précondensation ou d'une prépolymérisation d'au moins un diisocyanate élémentaire, lequel diisocyanate élémentaire présente au moins une fonction isocyanate aliphatique,
et par le fait qu'il comporte au plus 5 % de fonction isocyanate libre résiduelle, mesuré par dosage à la dibutylamine.

2. Polyisocyanate masqué selon la revendication 1, caractérisé par le fait qu'il comporte au moins une fonction isocyanate masquée selon l'invention reliée au squelette par l'intermédiaire d'un carbone de type sp³ portant un atome d'hydrogène, de préférence deux.

3. Polyisocyanate masqué selon l'une des revendications 1 et 2, caractérisé par le fait que ledit diisocyanate élémentaire est un polyméthylène diisocyanate.

4. Polyisocyanate masqué selon l'une des revendications 1 à 3, caractérisé par le fait que ledit polyisocyanate est un dérivé de condensation et/ou de di- et trimérisation d'un ou plusieurs diisocyanates élémentaires aliphatiques.

5. Polyisocyanate masqué selon l'une des revendications 1 à 4, caractérisé par le fait que ledit polyisocyanate est un composé de type biuret ou trimère d'un ou plusieurs diisocyanates aliphatiques.

6. Polyisocyanate masqué selon l'une des revendications 1 à 5 caractérisé par le fait que ledit polyisocyanate est un dérivé de trimérisation d'un polyméthylène diisocyanate.

7. Polyisocyanate masqué selon l'une des revendications 1 à 6, caractérisé par le fait que ledit diisocyanate élémentaire aliphatique est héxaméthylène diisocyanate.

8. Polyisocyanate masqué selon l'une des revendications 1 à 7, caractérisé par le fait que ledit dérivé aromatique hydroxylé répond à la formule (I)
Ar(R)ₙ(Y-Z)ₘ(OH)ₚ (I)
- où Ar est un reste aromatique ;
- où R représente un substituant hydrocarboné, en général alcoyle ;
- où Z est choisi parmi les fonctions nitriles et groupements carbonyles ;
- où Y est choisi parmi les groupements divalents, avantageusement une liaison simple,
-O-, -S-, NR'-,-CR'R"- avec R' et R" choisis parmi les radicaux hydrocarbonés, avantageusement alcoyle, de 1 à 6 atomes de carbone, avantageusement de 1 à 4, de préférence méthyle, plus préférentiellement hydrogène ;
- et où n + m + p est au plus égal au nombre de sommets substituables de Ar.

9. Polyisocyanate masqué selon l'une des revendications 8, caractérisé par le fait que p est égal à 1 et que m est avantageusement au plus égal à 2.

10. Polyisocyanate masqué selon l'une des revendications 8 et 9, caractérisé par le fait que Z est choisi parmi les groupements :
- alcoxycarbonyle ;
- amide ;
- alcoylcarbonyle ;
avec la condition que Z n'est pas porteuse d'hydrogène acide.

11. Polyisocyanate masqué selon l'une des revendications 8 à 10, caractérisé par le fait que la fonction hydroxyle n'est pas vicinale d'une ou de la fonction Z.

12. Polyisocyanate masqué selon l'une des revendications 8 à 11, caractérisé par le fait que Ar est choisi parmi les noyaux aromatiques à six chaînons carbonés ou azotés.

13. Polyisocyanate masqué selon l'une des revendications 1 à 12, caractérisé par le fait que ledit dérivé aromatique hydroxylé est un dérivé de l'un des acides para- ou métahydroxybenzoïque.

14. Polyisocyanate masqué selon l'une des revendications 1 à 13, caractérisé par le fait que ledit dérivé aromatique hydroxylé est l'acide para- ou métahydroxybenzoïque.

15. Polyisocyanate masqué selon l'une des revendications 12 à 14, caractérisé par le fait que ledit dérivé hydroxylé aromatique est le parahydroxybenzonitrile.

16. Polyisocyanate masqué selon l'une des revendications 1 à 15, caractérisé par le fait que son point de fusion apparent est au moins égal à 50°C.

17. Composition de poudres, notamment pour revêtement, caractérisée par le fait qu'elle comporte au moins un des polyisocyanates masqués, selon l'une des revendications 1 à 14, ledit polyisocyanate masqué étant sous la forme d'une poudre.

18. Composition selon la revendication 15, caractérisée par le fait qu'elle comporte en outre un catalyseur à base de zinc ou d'étain.

19. Composition selon les revendications 15 et 16, caractérisée par le fait qu'elle contient en outre une poudre de polyol.

20. Composition selon les revendications 17 à 19, caractérisée par le fait qu'elle comporte en outre un polyamide sous forme de poudre.

21. Procédé de synthèse d'un isocyanate masqué selon les revendications 1 à 16, caractérisé par le fait qu'il comporte la mise en contact dudit dérivé aromatique hydroxylé avec ledit isocyanate à une température au plus égale à 100°C.

22. Procédé selon la revendication 21, caractérisé par le fait qu'il comporte en outre l'étape de précipitation de l'isocyanate masqué au moyen de l'addition d'un solvant apolaire de type alcane ou alcène.

23. Procédé selon la revendication 22, caractérisé par le fait que le solvant apolaire de l'étape de précipitation est un alcane de C₄ à C₂₀.

24. Procédé selon l'une des revendications 22 à 23, caractérisé par le fait que ledit solvant est choisi de manière à ce que l'isocyanate soit soluble à hauteur d'au moins 50 grammes, avantageusement 100 grammes, de préférence 200 grammes par litre.

## Patentansprüche

1. Maskiertes Polyisocyanat, dessen Schmelzpunkt oder scheinbarer Schmelzpunkt in dem Falle von Verbindungen, die keinen gleichmäßigen Schmelzpunkt aufweisen, gemessen an einer Koffler-Bank oder mittels einer Kapillar-Methode, mindestens 30°C beträgt und dessen Glasübergangstemperatur mindestens 10°C beträgt,
dadurch gekennzeichnet, daß es erhalten werden kann durch die Kondensation in stöchiometrischem Überschuß von 0,5 bis 2%
- eines aromatischen, an dem Kern hydroxylierten Derivats, das eine elektronenanziehende Funktion, ausgewählt aus den carbonylhaltigen Funktionen und Nitrilfunktionen, trägt, mit der Maßgabe, daß eine direkte Bindung zwischen dem Kern und der elektronenanziehenden Funktion vorhanden ist, außer daß nicht bereits eine elektronenanziehende Gruppe an dem Kern vorhanden ist oder daß dieser heterocyclisch mit sechs Kettengliedern ist, und mit der Maßgabe, daß, wenn die carbonylhaltige Funktion eine Esterfunktion ist, deren Alkylteil eine Anzahl von Kohlenstoffatomen von höchstens 6 aufweist und verzweigt ist, wenn seine Anzahl von Kohlenstoffatomen 2 übersteigt;
- mit einem Polyisocyanat, das mehr als zwei Isocyanatfunktionen aufweist, aus einer Vorkondensation oder einer Präpolymerisation von mindestens einem grundlegenden Diisocyanat stammt, wobei dieses grundlegende Diisocyanat mindestens eine aliphatische Isocyanatfunktion aufweist, und dadurch, daß es höchstens 5% freie restliche Isocyanatfunktion, gemessen durch Bestimmung mittels Dibutylamin, aufweist.

2. Maskiertes Polyisocyanat nach Anspruch 1, dadurch gekennzeichnet, daß es mindestens eine erfindungsgemäß maskierte Isocyanatfunktion umfaßt, die an das Gerüst über das Zwischenglied eines sp³-Kohlenstoffs, der ein Wasserstoffatom, vorzugsweise zwei Wasserstoffatome trägt, gebunden ist.

3. Maskiertes Polyisocyanat nach einem der Ansprüche 1 und 2, dadurch gekennzeichnet, daß das grundlegende Diisocyanat ein Polymethylendiisocyanat ist.

4. Maskiertes Polyisocyanat nach einem der Ansprüche 1 bis 3, dadurch gekennzeichnet, daß das Polyisocyanat ein Derivat aus einer Kondensation und/oder einer Di- und Trimerisierung eines oder mehrerer grundlegender aliphatischer Diisocyanate ist.

5. Maskiertes Polyisocyanat nach einem der Ansprüche 1 bis 4, dadurch gekennzeichnet, daß das Polyisocyanat eine Verbindung vom Typ Biuret oder vom Typ Trimer eines oder mehrerer aliphatischer Diisocyanate ist.

6. Maskiertes Polyisocyanat nach einem der Ansprüche 1 bis 5, dadurch gekennzeichnet, daß das Polyisocyanat ein Derivat aus einer Trimerisierung eines Polymethylendiisocyanats ist.

7. Maskiertes Polyisocyanat nach einem der Ansprüche 1 bis 6, dadurch gekennzeichnet, daß das grundlegende aliphatische Diisocyanat Hexamethylendiisocyanat ist.

8. Maskiertes Polyisocyanat nach einem der Ansprüche 1 bis 7, dadurch gekennzeichnet, daß das aromatische hydroxylierte Derivat der Formel (I) entspricht
Ar(R)ₙ(Y-Z)ₘ(OH)ₚ (I)
- wo Ar ein aromatischer Rest ist,
- wo R für einen kohlenwasserstoffhaltigen Substituenten, im allgemeinen Alkyl, steht,
- wo Z ausgewählt ist aus den Nitrilfunktionen und carbonylhaltigen Gruppen,
- wo Y ausgewählt ist aus den zweiwertigen Gruppen, vorteilhafterweise einer Einfachbindung, -O-, -S-, -NR'-, -CR'R''-, wobei R' und R'' ausgewählt sind aus den kohlenwasserstoffhaltigen Resten, vorteilhafterweise Alkylresten, mit 1 bis 6, vorteilhafterweise 1 bis 4 Kohlenstoffatomen, vorzugsweise Methyl, mehr bevorzugt Wasserstoff,
- und wo n + m + p höchstens gleich der Anzahl der Maximalzahl von substituierbaren Positionen von Ar ist.

9. Maskiertes Polyisocyanat nach Anspruch 8, dadurch gekennzeichnet, daß p 1 ist und daß m vorteilhafterweise höchstens 2 ist.

10. Maskiertes Polyisocyanat nach einem der Ansprüche 8 und 9, dadurch gekennzeichnet, daß Z ausgewählt ist aus den Gruppen:
- Alkoxycarbonyl,
- Amid,
- Alkylcarbonyl,
mit der Maßgabe, daß Z kein saures Wasserstoffatom trägt.

11. Maskiertes Polyisocyanat nach einem der Ansprüche 8 bis 10, dadurch gekennzeichnet, daß die Hydroxylfunktion nicht zu einer oder der Funktion Z vizinal ist.

12. Maskiertes Polyisocyanat nach einem der Ansprüche 8 bis 11, dadurch gekennzeichnet, daß Ar aus den aromatischen Kernen mit sechs Kohlenstoff- oder Stickstoff-Kettengliedern ausgewählt ist.

13. Maskiertes Polyisocyanat nach einem der Ansprüche 1 bis 12, dadurch gekennzeichnet, daß das aromatische hydroxylierte Derivat ein Derivat einer der p- oder m-Hydroxybenzoesäuren ist.

14. Maskiertes Polyisocyanat nach einem der Ansprüche 1 bis 13, dadurch gekennzeichnet, daß das aromatische hydroxylierte Derivat p- oder m-Hydroxybenzoesäure ist.

15. Maskiertes Polyisocyanat nach einem der Ansprüche 12 bis 14, dadurch gekennzeichnet, daß das hydroxylierte aromatische Derivat p-Hydroxybenzonitril ist.

16. Maskiertes Polyisocyanat nach einem der Ansprüche 1 bis 15, dadurch gekennzeichnet, daß sein scheinbarer Schmelzpunkt mindestens 50°C beträgt.

17. Zusammensetzung von Pulvern, insbesondere für Überzüge, dadurch gekennzeichnet, daß sie mindestens eines der maskierten Polyisocyanate nach einem der Ansprüche 1 bis 14 umfaßt, wobei das maskierte Polyisocyanat in Form eines Pulvers vorliegt.

18. Zusammensetzung nach Anspruch 15, dadurch gekennzeichnet, daß sie darüber hinaus einen Katalysator auf Basis von Zink oder Zinn umfaßt.

19. Zusammensetzung nach den Ansprüchen 15 und 16, dadurch gekennzeichnet, daß sie darüber hinaus ein Polyol-Pulver enthält.

20. Zusammensetzung nach den Ansprüchen 17 bis 19, dadurch gekennzeichnet, daß sie darüber hinaus ein Polyamid in Pulverform umfaßt.

21. Verfahren zur Synthese eines maskierten Isocyanats nach den Ansprüchen 1 bis 16, dadurch gekennzeichnet, daß es umfaßt, das aromatische hydroxylierte Derivat mit dem Isocyanat bei einer Temperatur von höchstens 100°C in Berührung zu bringen.

22. Verfahren nach Anspruch 21, dadurch gekennzeichnet, daß es darüber hinaus den Schritt einer Präzipitation des maskierten Isocyanats mittels der Zugabe eines apolaren Lösemittels vom Alkan- oder Alkentyp umfaßt.

23. Verfahren nach Anspruch 22, dadurch gekennzeichnet, daß das apolare Lösemittel des Präzipitationsschritts ein C₄-bis C₂₀-Alkan ist.

24. Verfahren nach einem der Ansprüche 22 bis 23, dadurch gekennzeichnet, daß das Lösemittel so ausgewählt wird, daß das Isocyanat bis zu einer Höhe von mindestens 50 g, vorteilhafterweise 100 g, vorzugsweise 200 g pro Liter löslich ist.

## Claims

1. Masked polyisocyanate whose melting point, or apparent melting point in the case of compounds which do not have a sharp melting point, measured with a Koffler machine or using a method of capillary type, is at least equal to 30°C, and whose glass transition temperature is at least equal to 10°C, characterized in that it can be obtained by the condensation, with a 0.5 to 2% stoichiometric excess,
• of an aromatic derivative hydroxylated on the ring and bearing an electron-withdrawing group chosen from carbonyl and nitrile functions, with the condition that there is a direct bond between the ring and the elctron-withdrawing function, unless there is already an electron-withdrawing group on the ring, or that this ring is a six-membered heterocyclic ring, and with the condition that when the carbonyl function is an ester function, its alkyl portion contains not more than 6 carbon atoms and is branched when its carbon number exceeds 2;
• with a polyisocyanate containing more than two isocyanate functions, derived from a precondensation or prepolymerization of at least one elemental diisocyanate, this elemental diisocyanate containing at least one aliphatic isocyanate function;
and in that it comprises not more than 5% of residual free isocyanate function, measured by assay with dibutylamine.

2. Masked polyisocyanate according to Claim 1, characterized in that it comprises at least one masked isocyanate function according to the invention, linked to the skeleton via a carbon of sp³ type bearing a hydrogen atom, preferably two.

3. Masked polyisocyanate according to either of Claims 1 and 2, characterized in that the said elemental diisocyanate is a polymethylene diisocyanate.

4. Masked polyisocyanate according to one of Claims 1 to 3, characterized in that the said polyisocyanate is a condensation and/or di- and trimerization derivative of one or more aliphatic elemental diisocyanates.

5. Masked polyisocyanate according to one of Claims 1 to 4, characterized in that the said polyisocyanate is a compound of biuret type or a trimer of one or more aliphatic diisocyanates.

6. Masked polyisocyanate according to one of Claims 1 to 5, characterized in that the said polyisocyanate is a crimerization derivative of a polymethylene diisocyanate.

7. Masked polyisocyanate according to one of Claims 1 to 6, characterized in that the said aliphatic elemental diisocyanate is hexamethylene diisocyanate.

8. Masked polyisocyanate according to one of Claims 1 to 7, characterized in that the said hydroxylated aromatic derivative corresponds to the formula (I)
Ar(R)ₙ(Y-Z)ₘ(OH)ₚ (I)
where Ar is an aromatic residue;
where R represents a hydrocarbon substituent, generally an alkyl substituent;
where Z is chosen from nitrile functions and carbonyl groups;
where Y is chosen from divalent groups, advantageously a single bond, -O-, -S-, NR'-, -CR'R"- with R' and R" chosen from hydrocarbon radicals which are advantageously alkyl, of 1 to 6 carbon atoms, advantageously of 1 to 4, preferably methyl, more preferably hydrogen;
and where n + m + p is not more than the number of substitutable ring members of Ar.

9. Masked polyisocyanate according to Claim 8, characterized in that p is equal to 1 and that m is advantageously not more than 2.

10. Masked polyisocyanate according to either of Claims 8 and 9, characterized in that Z is chosen from the groups:
- alkoxycarbonyl;
- amide;
- alkylcarbonyl;
on condition that Z does not bear an acidic hydrogen.

11. Masked polyisocyanate according to one of Claims 8 to 10, characterized in that the hydroxyl function is not vicinal to a or to the function Z.

12. Masked polyisocyanate according to one of Claims 8 to 11, characterized in that Ar is chosen from carbon- or nitrogen-based six-membered aromatic rings.

13. Masked polyisocyanate according to one of Claims 1 to 12, characterized in that the said hydroxylated aromatic derivative is a derivative of one of the para- or meta-hydroxybenzoic acids.

14. Masked polyisocyanate according co one of Claims 1 to 13, characterized in that the said hydroxylated aromatic derivative is para- or meta-hydroxybenzoic acid.

15. Masked polyisocyanate according to one of Claims 12 to 14, characterized in that the said aromatic hydroxylated derivative is para-hydroxybenzonitrile.

16. Masked polyisocyanate according to one of Claims 1 to 15, characterized in that its apparent melting point is at least equal to 50°C.

17. Powder composition, especially for coating, characterized in that it includes at least one of the masked polyisocyanates according to one of Claims 1 to 14, the said masked polyisocyanate being in the form of a powder.

18. Composition according to Claim 15, characterized in that it additionally includes a catalyst based on zinc or tin.

19. Composition according to Claims 15 and 16, characterized in that it additionally contains a polyol powder.

20. Composition according to Claims 17 to 19, characterized in that it additionally includes a polyamide in powder form.

21. Process for the synthesis of a masked isocyanate according to Claims 1 to 16, characterized in that it includes the placing in contact of the said hydroxylated aromatic derivative with the said isocyanate at a temperature of not more than 100°C.

22. Process according to Claim 21, characterized in that it additionally includes the step of precipitation of the masked isocyanate by means of the addition of an apolar solvent of alkane or alkene type.

23. Process according to Claim 22, characterized in that the apolar solvent of the precipitation step is a C₄ to C₂₀ alkane.

24. Process according to one of Claims 22 to 23, characterized in that the said solvent is chosen such that the isocyanate is soluble to an extent of at least 50 grams, advantageously 100 grams and preferably 200 grams per litre.
